# EUROPÄISCHE PATENTSCHRIFT

(11) **EP 0 891 322 B1**
(45) Veröffentlichungstag und Bekanntmachung des Hinweises auf die Patenterteilung: **27.12.2000**
(21) Anmeldenummer: 97914255.1
(22) Anmeldetag: 17.03.1997
(51) Int. Cl.: C07C 69/753, C07C 67/343, A61K 31/215

(54) **DIMETHYL-SUBSTITUIERTE CYCLOHEXANDIENDERIVATE**
DIMETHYL-SUBSTITUTED CYCLOHEXANE DIENE DERIVATIVES
DERIVES DU CYCLOHEXANEDIENE DIMETHYLE SUBSTITUES

(30) Priorität: 29.03.1996 DE 19612645
(43) Veröffentlichungstag der Anmeldung: 20.01.1999
(73) Patentinhaber: BAYER AG, 51368 Leverkusen (DE)
(72) Erfinder: URBAHNS, Klaus, D-42115 Wuppertal (DE); MAULER, Frank, D-51491 Overath (DE)
(86) Internationale Anmeldenummer: EP9701328
(87) Internationale Veröffentlichungsnummer: WO9736853

(56) Entgegenhaltungen:
- CHEMICAL AND PHARMACEUTICAL BULLETIN, Bd. 39, Nr. 11, November 1991, TOKYO JP, Seiten 2915-2923, XP002033122 KAZUHIKO TAKE ET AL.: "Agents for the Treatment of Overactive Detrusor.I. Synthesis and Structure-Activity Relationships of 1,1'-Biphenyl Derivatives" in der Anmeldung erwähnt

## Beschreibung

Die vorliegende Erfindung betrifft Dimethyl-substituierte Cyclohexandienderivate, ein Verfahren zu ihrer Herstellung und ihre Verwendung als Arzneimittel.

Es ist bereits bekannt, daß 1,1'-Biphenyl-2,6'-dicarbonsäure Derivate eine Muskelkontraktion inhibierende Wirkung besitzen [vgl. hierzu Chem. Pharm. Bull., 39 (11), 2915 - 23, 1991; GB 87-18906 870810 / GB 87-19441 870817].

Die Erfindung betrifft Dimethyl-substituierte Cyclohexandienderivate der allgemeinen Formeln (Ia,b) 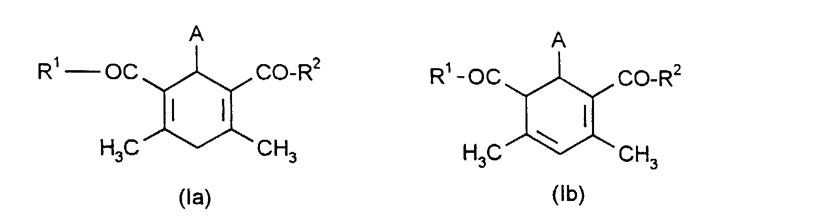 in welcher
- A: für Cycloalkyl mit 3 bis 6 Kohlenstoffatomen oder für Aryl mit 6 bis 10 Kohlenstoffatomen steht, die gegebenenfalls bis zu 3-fach gleich oder verschieden durch Nitro, Cyano, Cycloalkyl mit 3 bis 7 Kohlenstoffatomen, Halogen, Trifluormethyl oder durch geradkettiges oder verzweigtes Alkylthio, Alkyl oder Alkoxy mit jeweils bis zu 6 Kohlenstoffatomen substituiert sind,
- R¹ und R²: gleich oder verschieden sind und
für Wasserstoff oder geradkettiges oder verzweigtes Alkyl oder Alkoxy mit jeweils bis zu 8 Kohlenstoffatomen, Hydroxy oder für eine Gruppe der Formel -NR³R⁴ stehen,
worin
R³ und R⁴ gleich oder verschieden sind und
Wasserstoff, Aryl mit 6 bis 10 Kohlenstoffatomen oder geradkettiges oder verzweigtes Alkyl mit bis zu 4 Kohlenstoffatomen bedeuten,
und deren Salze.

Bevorzugte Salze sind physiologisch unbedenkliche Salze. Dies sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (Ia,b),
in welcher
- A: für Cyclohexyl, Phenyl oder Naphthyl steht, die gegebenenfalls bis zu 3-fach gleich oder verschieden durch Nitro, Cyano, Fluor, Chlor, Brom, Iod, Cyclopentyl, Cyclohexyl, Trifluormethyl oder durch geradkettiges oder verzweigtes Alkylthio, Alkyl oder Alkoxy mit jeweils bis zu 4 Kohlenstoffatomen substituiert sind,
- R¹ und R²: gleich oder verschieden sind und
für Wasserstoff oder geradkettiges oder verzweigtes Alkyl oder Alkoxy mit jeweils bis zu 6 Kohlenstoffatomen, Hydroxy oder für eine Gruppe der Formel -NR³R⁴ stehen,
worin
R³ und R⁴ gleich oder verschieden sind und
Wasserstoff, Phenyl oder geradkettiges oder verzweigtes Alkyl mit bis zu 3 Kohlenstoffatomen bedeuten,
und deren Salze.

Besonders bevorzugt sind Verbindungen der allgemeinen Formel (Ia,b),
in welcher
- A: für Cyclohexyl oder Phenyl steht, die gegebenenfalls bis zu 3-fach gleich oder verschieden durch Nitro, Cyano, Fluor, Chlor, Brom, Iod, Trifluormethyl, Cyclohexyl, Methyl, Methoxy oder durch Methylthio substituiert sind,
- R¹ und R²: gleich oder verschieden sind und
für Wasserstoff oder geradkettiges oder verzweigtes Alkyl oder Alkoxy mit jeweils bis zu 5 Kohlenstoffatomen, Hydroxy oder für eine Gruppe der Formel -NR³R⁴ stehen,
worin
- R³ und R⁴: gleich oder verschieden sind und
Wasserstoff, Phenyl, Methyl oder Ethyl bedeuten,
und deren Salze.

Außerdem wurde ein Verfahren zur Herstellung der erfindungsgemäßen Verbindungen der allgemeinen Formeln (Ia) und (Ib) gefunden, dadurch gekennzeichnet, daß man

Verbindungen der allgemeinen Formel (II) 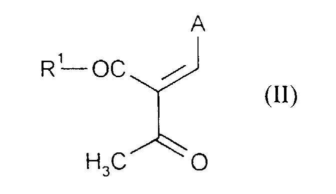 in welcher
A und R¹ die oben angegebenen Bedeutungen haben,
mit Verbindungen der allgemeinen Formel (III) 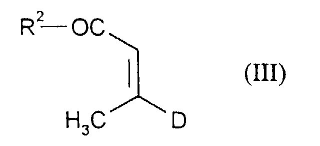 in welcher
- R²: die oben angegebenen Bedeutungen hat,
- D: für einen Rest der Formel

CH₂―P(C₆H₅)₃⁺ E⁻

oder

CH₂-P(O)(OR⁵)(OR⁶)

steht,
worin
R⁵ und R⁶ gleich oder verschieden sind und geradkettiges oder verzweigtes Alkyl mit bis zu 4 Kohlenstoffatomen bedeuten,
E für Chlorid oder Bromid, vorzugsweise für Bromid steht,
in inerten Lösemitteln, in Anwesenheit einer Base umsetzt und im Fall der Säuren R¹/R² ≠ OH die Ester nach üblichen Methoden hydrolysiert,
und im Fall der Amide -CO-NH₂, ausgehend von den Säuren mit Ammoniak oder den entsprechenden Aminen (NHR³R⁴) umsetzt,
oder ausgehend von den entsprechenden Estern der Cyclohexandienderivate der allgemeinen Formel (la,b) eine Aluminiumtrimethylvermittelte Aminolyse durch die Amine (NHR³R⁴) oder deren Hydrochloride durchführt.

Die anfallenden Doppelbindungsisomere können durch Chromatographie und/oder Kristallisation getrennt werden.

Das erfindungsgemäße Verfahren kann durch folgendes Formelschema beispielhaft erläutert werden:

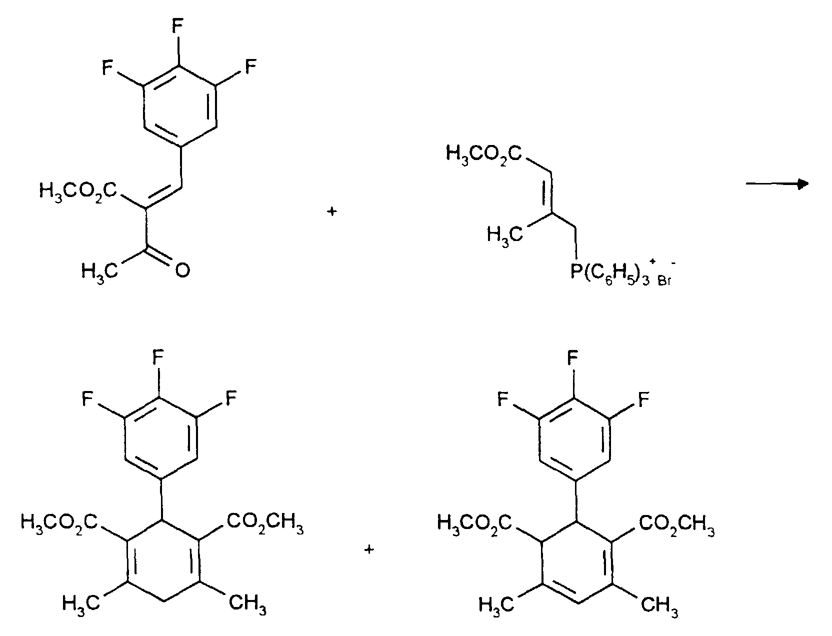

Als Lösemittel eignen sich alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder halogenierte Kohlenwasserstoffe wie Methylenchlorid, Tetrachlorkohlenstoff oder Kohlenwasserstoffe wie Benzol oder Toluol, oder Pyridin. Ebenso ist es möglich, Gemische der genannten Lösemittel zu verwenden. Bevorzugt ist Methanol.

Als Basen eignen sich anorganische oder organische Basen. Hierzu gehören vorzugsweise Alkalihydroxide wie z.B. Natriumhydroxid oder Kaliumhydroxid, Erdalkalihydroxide wie z.B. Bariumhydroxid, Alkalicarbonate wie Natriumcarbonat oder Kaliumcarbonat, Erdalkalicarbonate wie Calciumcarbonat, oder Alkalialkoholate wie beispielsweise Natriummethanolat oder Natriumethanolat oder Triethylamine oder 1,8-Diazabicyclo[5.4,0]undec-7-en (DBU). Bevorzugt ist Natriummethanolat.

Die Base wird im allgemeinen in einer Menge von 0,1 mol bis 100 mol, bevorzugt von 1 mol bis 10 mol bezogen auf 1 mol der Verbindungen der allgemeinen Formel (II) eingesetzt.

Die Reaktionstemperaturen können in einem größeren Bereich variiert werden. Im allgemeinen arbeitet man zwischen +10°C und +150°C, vorzugsweise zwischen +20°C und +100°C, insbesondere bei Raumtemperatur.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid Besonders bevorzugt werden Alkohole wie Methanol, Ethanol, Propanol oder Isopropanol verwendet. Ebenso ist es möglich, Gemische der genannten Lösemittel einzusetzen

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Als Lösemittel für die Amidierung eignen sich die oben aufgeführten organischen Lösemittel, die sich unter den Reaktionsbedingungen nicht verändern, wobei die Ether bevorzugt sind. Besonders bevorzugt ist Tetrahydrofuran.

Als Hilfsmittel für die Amidierung mit den Verbindungen der Formel (IV) eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexylfluorophosphat oder Phosphonsäurediphenylesteramid oder Methansulfonsäurechlorid oder Thionylchlorid, Trifluoressigsäureanhydrid, gegebenenfalls in Anwesenheit von Basen wie Triethylamin, Pyridin oder N-Ethylmorpholin oder N-Methylpiperidin oder Dicyclohexylcarbodiimid und N-Hydroxysuccinimid, Alkoxycarbonylsulfonyltrialkylammoniumhydroxide, Acetanhydrid/NaOAc/Phosphorsäure/Mineralsäuren wie beispielsweise Schwefelsäure oder organische Sulfonsäuren wie beispielsweise p-Toluol-sulfonsäure. Bevorzugt ist Thionylchlorid/Pyridin.

Als Hilfsmittel eignen sich für die Amidierung im allgemeinen organische Sulfonsäuren, wie p-Toluolsulfonsäure oder wasserfreie Mineralsäure wie Phosphorsäure oder Schwefelsäure. Bevorzugt ist p-Toluolsulfonsäure-Hydrat.

Das Hilfsmittel wird in einer Menge von 0,1 mol bis 1 mol, bevorzugt von 0,1 mol bis 0,2 mol jeweils bezogen auf 1 mol der Verbindungen der zu amidierenden Verbindungen eingesetzt.

Die Aluminiumtrimethylvermittelte Aminolyse wird in einem der oben aufgeführten Lösemittel, vorzugsweise in Toluol bei Rückflußtemperatur durchgeführt.

Die Amidierung kann bei Normaldruck, aber auch bei erhöhtem oder erniedrigtem Druck (z.B. 0,5 bis 3 bar) durchgeführt werden. Im allgemeinen arbeitet man bei Normaldruck.

Die Verbindungen der allgemeinen Formel (III) sind an sich bekannt oder nach üblichen Methoden herstellbar.

Die Verbindungen der allgemeinen Formel (II) sind bekannt oder können beispielsweise hergestellt werden, indem man,

Aldehyde der allgemeinen Formel (IV)

A-CHO (IV)

in welcher
- A: die oben angegebenen Bedeutungen hat,
mit Verbindungen der allgemeinen Formel (V)

H₃C-CO-CH₂-CO-R¹ (V)

in welcher
- R¹: die oben angegebenen Bedeutungen hat,
in einem organischen Lösemittel und in Anwesenheit einer Base umsetzt.

Als Lösemittel eignen sich alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Besonders bevorzugt sind Ethanol und Methanol.

Als Basen eignen sich im allgemeinen Alkalihydride- oder alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt ist Piperidin.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Verbindungen der allgemeinen Formel (IV) und (V) sind an sich bekannt oder können nach üblichen Methoden hergestellt werden.

Die erfindungsgemäßen Verbindungen der allgemeinen Formel (Ia,b) zeigen ein nicht vorhersehbares, wertvolles pharmakologisches Wirkspektrum.

Sie sind Modulatoren mit Selektivität für Charybdotoxin-sensitive, calciumabhängige Kalium-Kanäle (IK(Ca)-Kanäle), insbesondere des zentralen Nervensystems.

Aufgrund ihrer pharmakologischen Eigenschaften können sie für die Herstellung von Arzneimitteln zur Behandlung von zentral degenerativen Erkrankungen eingesetzt werden, bei Auftreten von Demenzen wie Multiinfarktdemenz (MID), primär degenerativer Demenz (PDD), präseniler und seniler Demenz vom Typ der Alzheimerschen Krankheit, HIV-Demenz und andere Demenzformen, zur Behandlung der Parkinsonschen Krankheit, amyotrophischer Lateralsklerose sowie multipler Sklerose, Krebs, Restenose und Sichelzellanämie.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe und Bekämpfung der Folgen cerebraler Durchblutungsstörungen wie cerebraler Ischämien, Schlaganfällen, Schädel-Hirn-Traumen und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüberhinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Bluthochdruck, Arrhythmie, Angina und Diabetes.

### Rubidium-Efflux aus C6-BU1-Glioma-Zellen

Die Versuche wurden mit geringfügigen Veränderungen entsprechend der von Tas et al. (Neurosci. Lett. 94, 279-284, (1988)) beschriebenen Methode durchgeführt Dazu werden Ratten C6-BU1-Glioma-Zellen verwendet. Aus den Daten wird die durch Ionomycin hervorgerufene Erhöhung des Effluxes über den Basalefflux berechnet und als 100 % gesetzt. Die Stimulationen in Gegenwart von Prüfsubstanzen werden dann auf diesen Wert bezogen. Die Detektion erfolgt über Atomabsorptionsspektroskopie.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formeln (Ia) und (Ib) enthalten, oder die aus einem oder mehreren Wirkstoffen der Formeln (Ia) und (Ib) bestehen, sowie Verfahren zur Herstellung dieser Zubereitungen.

Die Wirkstoffe der Formeln (Ia) und (Ib) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formeln (Ia) und (Ib) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formeln (Ia) und (Ib) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Herstellungsbeispiele

### Beispiel 1 und Beispiel 2

2-(4-Chloro-3-trifluoromethylphenyl)-4,6-dimethylcyclohexa-3,6-dien-1,5-dicarbonsäure-dimethylester (Beispiel 1) 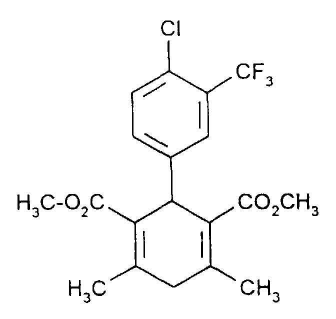 2-(4-Chloro-3-trifluoromethylphenyl)-4,6-dimethylcyclohexa-3,5-dien-1,5-dicarbonsäure-dimethylester (Beispiel 2) 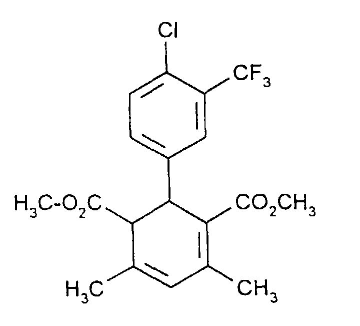 15,0 g (33 mmol) 3-Carboxymethyl-2-methyl-allyl-triphenylphosphoniumbromid werden in 300 ml trockenem Methanol gelöst und bei 0°C mit NaOCH₃ versetzt. Nach 1 h werden 9,2 g (0,033 mol) 2-Acetyl-3-(4-chlor-3-trifluormethylphenyl)propensäuremethylester zugesetzt. Man läßt 2 h bei Raumtemperatur stehen. Dann wird die Mischung mit Essigsäure angesäuert (pH = 5) und eingeengt. Nach Aufnahme in Methylenchlorid wäscht man mit H₂O, trocknet und engt erneut ein. Dann wird über MPLC (Methylenchlorid / Essigsäureethylester = 20:1) getrennt und man erhält 1,65 g des Beispiels 1 sowie 5,18 g des Beispiels 2.
Mp.: 91°C (Beispiel 1)
Mp.: 104°C (Beispiel 2)
In Analogie zur Vorschrift der Beispiele 1 und 2 werden die in den Tabellen 1 + 2 aufgeführten Verbindungen hergestellt:

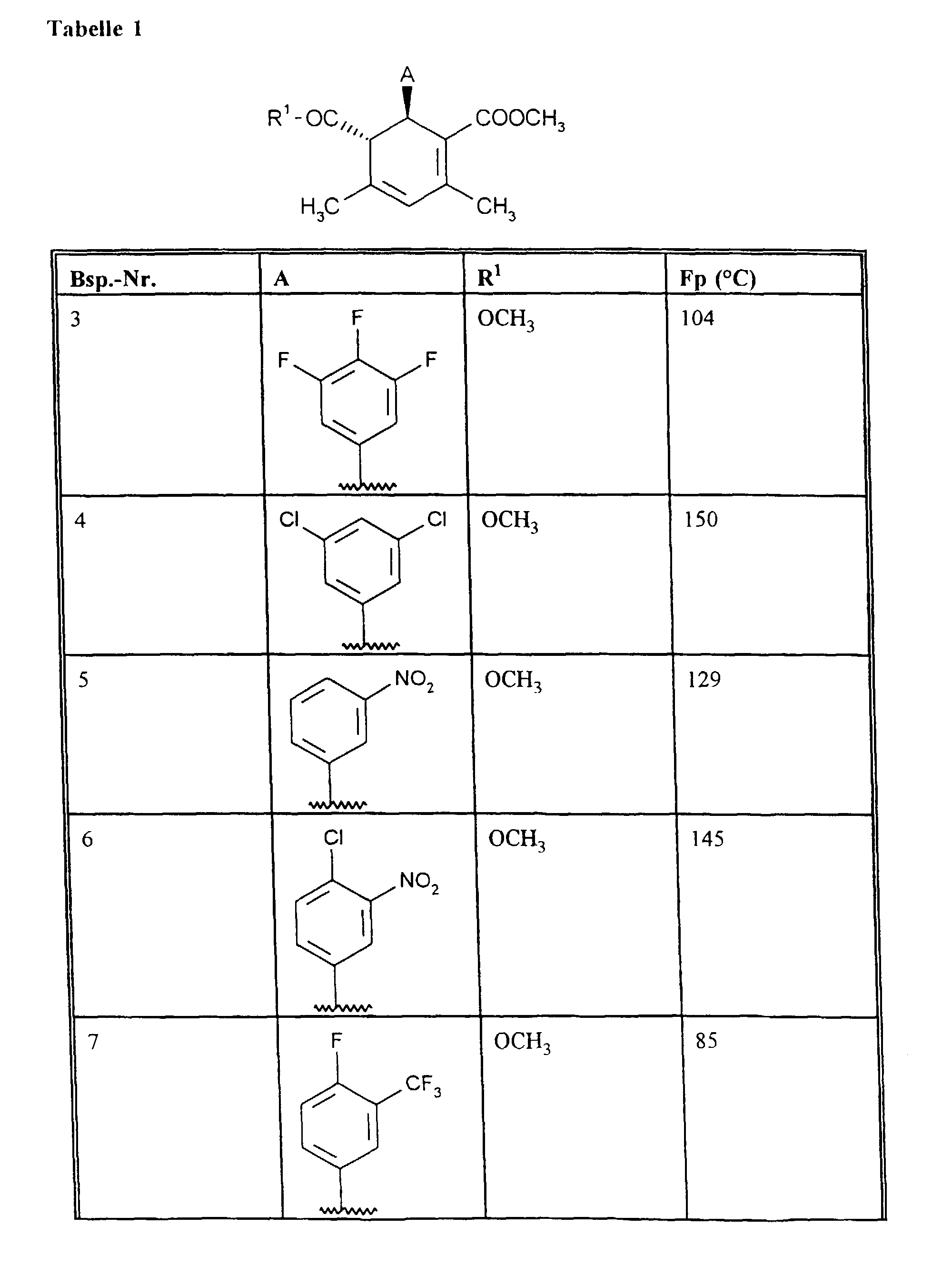

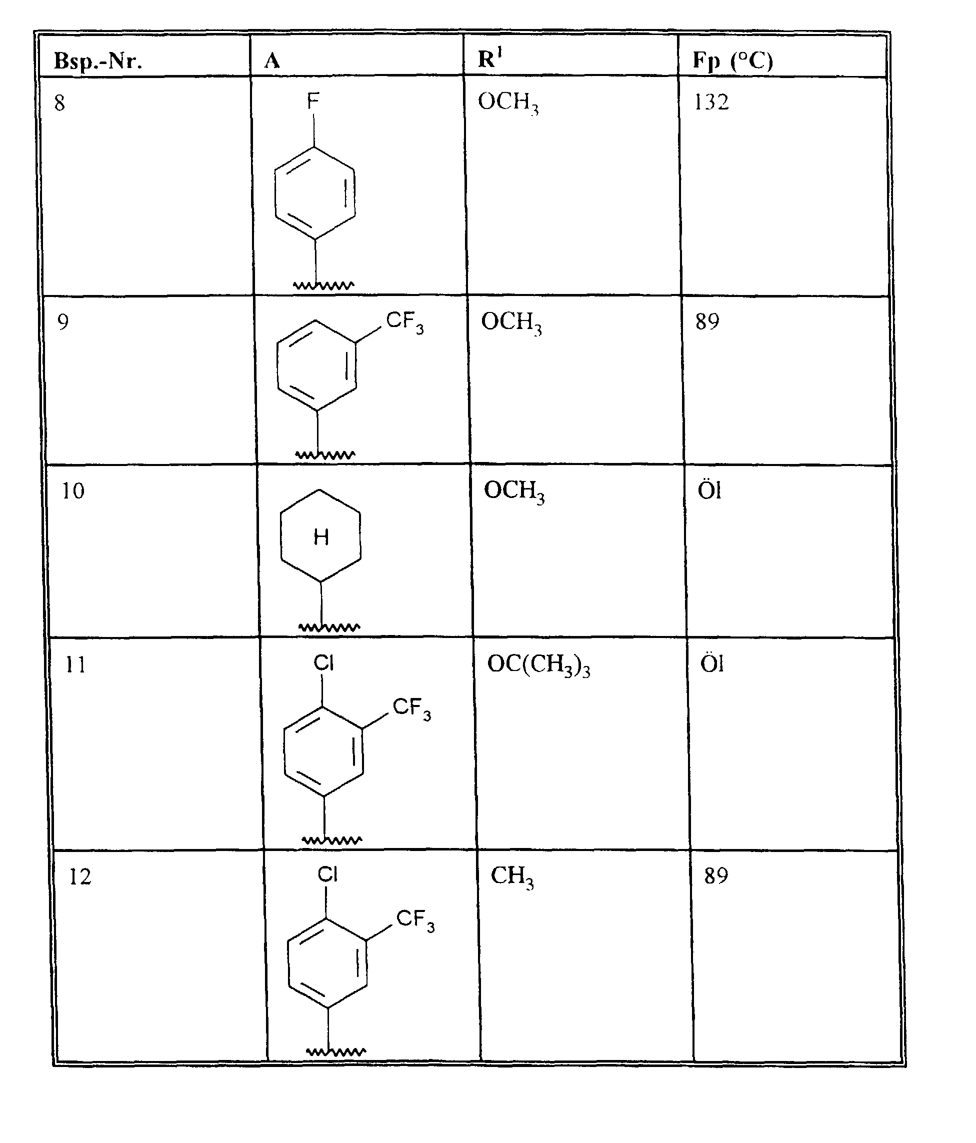

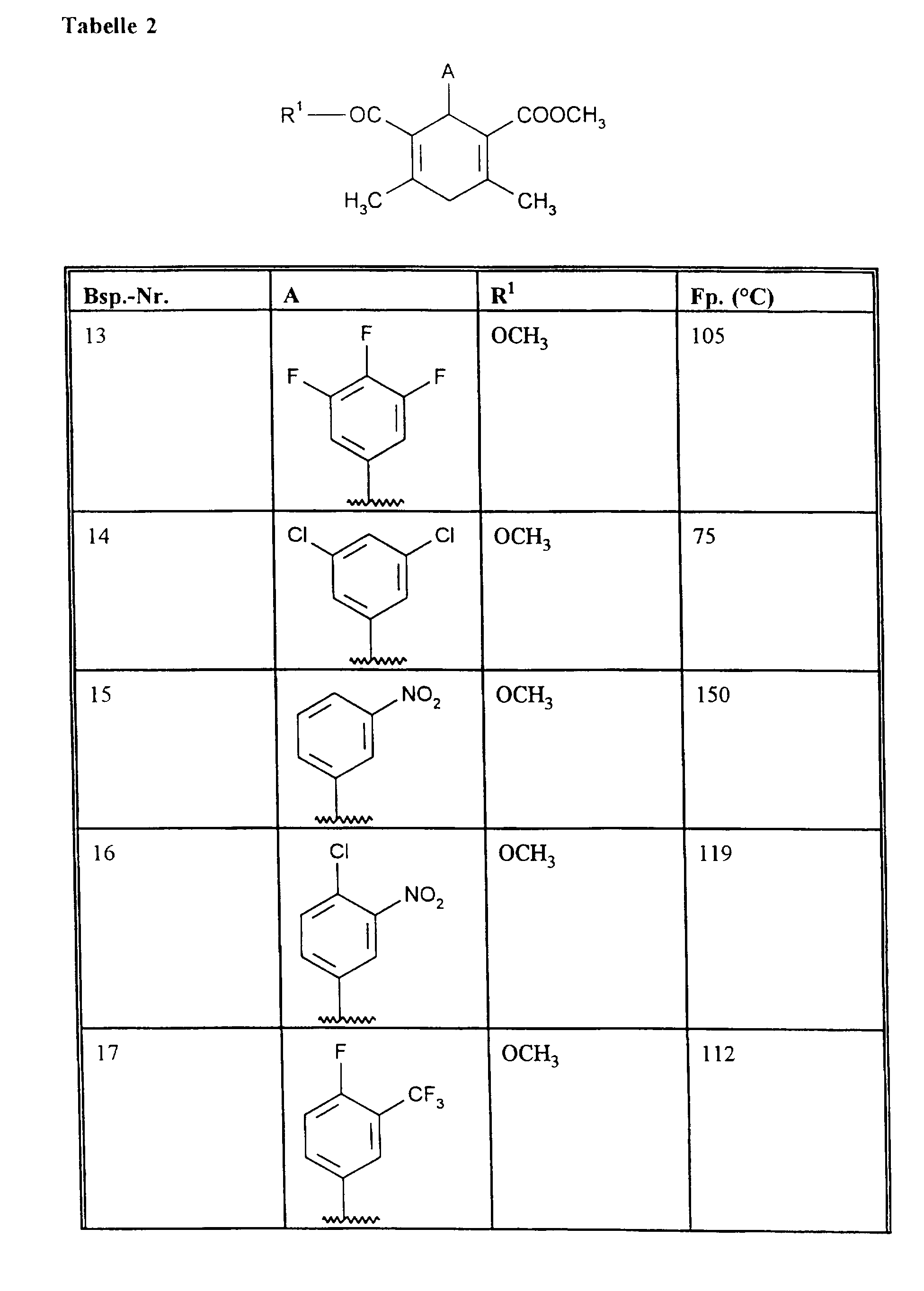

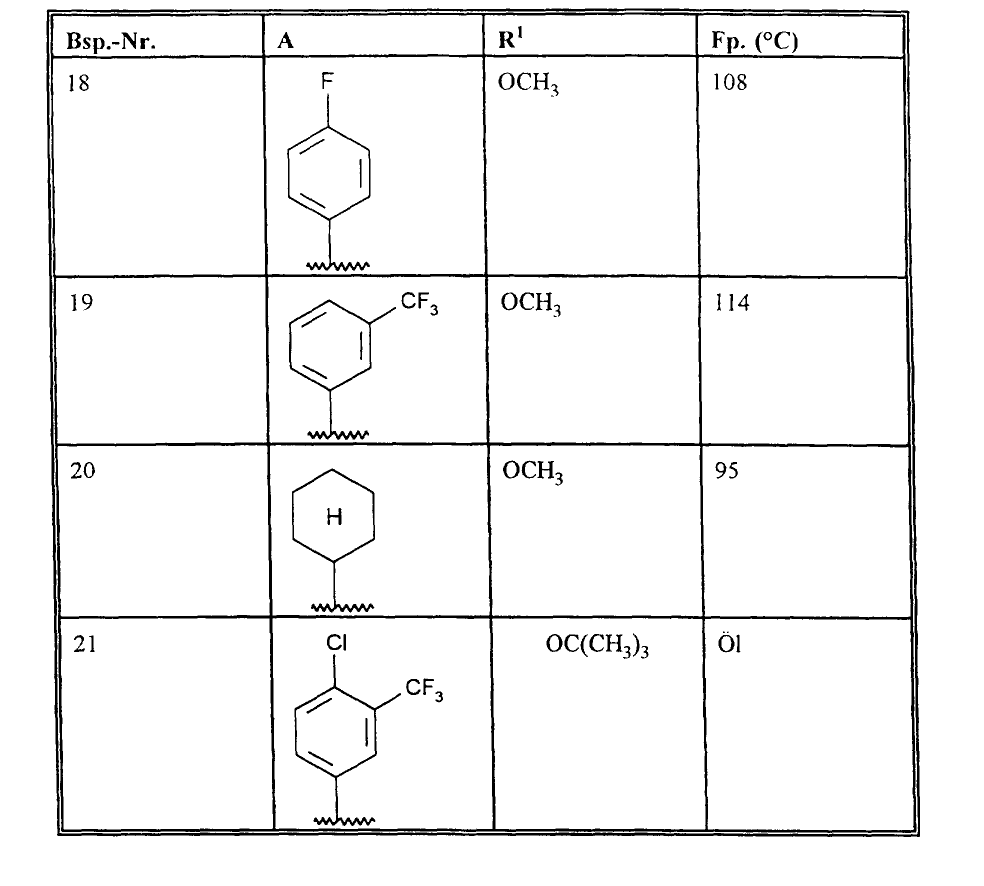

### Beispiel 22

4,6-Dimethyl-2-(4-chlor-3-trifluormethyl-phenyl)cyclohexa-3,6-dien-1,3-dicarbonsäure-monomethylester 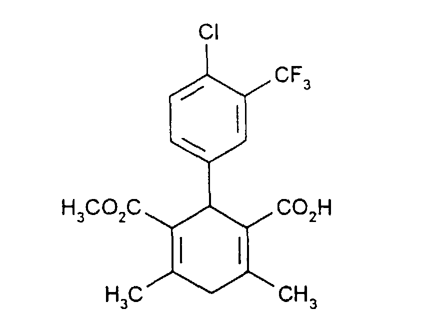 1,5 g (3,3 mmol) der Verbindung aus Beispiel 21 werden in 50 ml Methylenchlorid gelöst und mit 5 ml Trifluoressigsäure versetzt. Man rührt 3 h bei RT und erhält 0,86 g der Titelverbindung.
Fp.: 165°C

### Beispiel 23

5-Carbamoyl-2,4-dimethyl-6-(4-chlor-3-trifluormethyl-phenyl)cyclohexa-1,4-diencarbonsäure-methylester 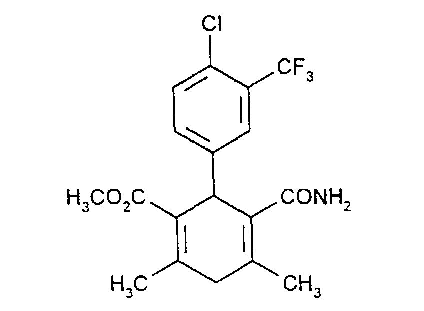 447 mg (1,15 mmol) der Verbindung aus Beispiel 22 werden mit SOCl₂ 2 h bei Rückfluß gerührt. Dann wird eingeengt und der Rückstand in 10 ml trockenem THF gelöst. Die Lösung tropft man in 20 ml NH₃-Wasser bei 0°C. Man destilliert das Filtrat weitgehend ab und extrahiert 3 x mit Essigsäureethylester Chromatographie des vereinigten Materials (Essigsäureethylester / Petrolether = 2:1)) liefert 152 mg der Titelverbindung.
Fp.: 153°C.

## Patentansprüche

1. Cyclohexandienderivate der allgemeinen Formel (Ia,b) 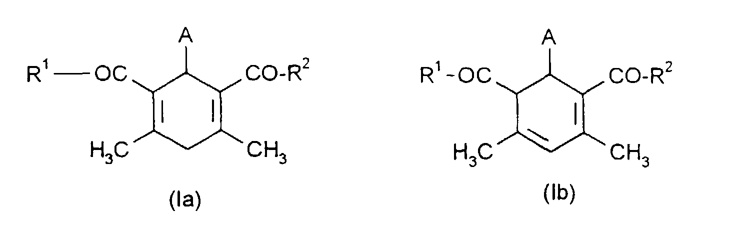 in welcher
A für Cycloalkyl mit 3 bis 6 Kohlenstoffatomen oder
für Aryl mit 6 bis 10 Kohlenstoffatomen steht, die gegebenenfalls bis zu 3-fach gleich oder verschieden durch Nitro, Cyano, Cycloalkyl mit 3 bis 7 Kohlenstoffatomen, Halogen, Trifluormethyl oder durch geradkettiges oder verzweigtes Alkylthio, Alkyl oder Alkoxy mit jeweils bis zu 6 Kohlenstoffatomen substituiert sind,
R¹ und R² gleich oder verschieden sind und
für Wasserstoff oder geradkettiges oder verzweigtes Alkyl oder Alkoxy mit jeweils bis zu 8 Kohlenstoffatomen, Hydroxy oder für eine Gruppe der Formel -NR³R⁴ stehen,
worin
R³ und R⁴ gleich oder verschieden sind und
Wasserstoff, Aryl mit 6 bis 10 Kohlenstoffatomen oder geradkettiges oder verzweigtes Alkyl mit bis zu 4 Kohlenstoffatomen bedeuten,
und deren Salze.

2. Cyclohexandienderivate nach Anspruch 1, dadurch gekennzeichnet, daß
A für Cyclohexyl, Phenyl oder Naphthyl steht, die gegebenenfalls bis zu 3-fach gleich oder verschieden durch Nitro, Cyano, Fluor, Chlor, Brom, lod, Cyclopentyl, Cyclohexyl, Trifluormethyl oder durch geradkettiges oder verzweigtes Alkylthio, Alkyl oder Alkoxy mit jeweils bis zu 4 Kohlenstoffatomen substituiert sind,
R¹ und R² gleich oder verschieden sind und
für Wasserstoff oder geradkettiges oder verzweigtes Alkyl oder Alkoxy mit jeweils bis zu 6 Kohlenstoffatomen, Hydroxy oder für eine Gruppe der Formel -NR³R⁴ stehen,
worin
R³ und R⁴ gleich oder verschieden sind und
Wasserstoff, Phenyl oder geradkettiges oder verzweigtes Alkyl mit bis zu 3 Kohlenstoffatomen bedeuten,
und deren Salze.

3. Cyclohexanderivate nach Anspruch 1, dadurch gekennzeichnet, daß
A für Cyclohexyl oder Phenyl steht, die gegebenenfalls bis zu 3-fach gleich oder verschieden durch Nitro, Cyano, Fluor, Chlor, Brom, Iod, Trifluormethyl, Cyclohexyl, Methyl, Methoxy oder durch Methylthio substituiert sind,
R¹ und R² gleich oder verschieden sind und
für Wasserstoff oder geradkettiges oder verzweigtes Alkyl oder Alkoxy mit jeweils bis zu 5 Kohlenstoffatomen, Hydroxy oder für eine Gruppe der Formel -NR³R⁴ stehen,
worin
R³ und R⁴ gleich oder verschieden sind und
Wasserstoff, Phenyl, Methyl oder Ethyl bedeuten,
und deren Salze.

4. Verfahren zur Herstellung von Cyclohexandienderivaten nach einem der Ansprüche 1 bis 3, dadurch gekennzeichnet, daß man Verbindungen der allgemeinen Formel (II) 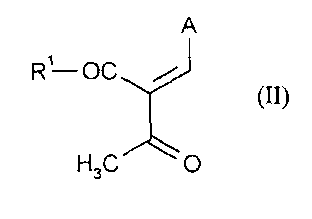 in welcher
A und R¹ die in den Ansprüchen 1 bis 3 angegebenen Bedeutungen haben,
mit Verbindungen der allgemeinen Formel (III) 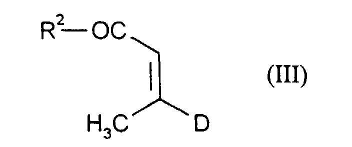 in welcher
R² die in den Ansprüchen 1 bis 3 angegebenen Bedeutungen hat,
D für einen Rest der Formel
CH₂―P(C₆H₅)₃⁺ E⁻
oder
CH₂-P(O)(OR⁵)(OR⁶)
steht,
worin
R⁵ und R⁶ gleich oder verschieden sind und geradkettiges oder verzweigtesAlkyl mit bis zu 4 Kohlenstoffatomen bedeuten,
E für Chlorid oder Bromid steht,
in inerten Lösemitteln, in Anwesenheit einer Base umsetzt und im Fall der Säuren R¹/R² ≠ OH die Ester hydrolysiert,
und im Fall der Amide -CO-NH₂, ausgehend von den Säuren mit Ammoniak oder den entsprechenden Aminen (NHR³R⁴) umsetzt,
oder ausgehend von den entsprechenden Estern der Cyclohexandienderivate der allgemeinen Formel (Ia,b) eine Aluminiumtrimethylvermittelte Aminolyse durch die Amine (NHR³R⁴) oder deren Hydrochloride durchführt.

5. Cyclohexandienderivate nach einem der Ansprüche 1 bis 3 zur therapeutischen Anwendung.

6. Arzneimittel enthaltend mindestens ein Cyclohexandienderivat nach einem der Ansprüche 1 bis 3.

7. Arzneimittel nach Anspruch 6 als Modulator mit Selektivität für Charybdotoxin-sensitive, calciumabhängige Kalium-Kanäle.

8. Verwendung der Cyclohexandienderivate nach einem der Ansprüche 1 bis 3 zur Herstellung von Arzneimitteln.

9. Verwendung nach Anspruch 8 zur Herstellung von Modulatoren mit Selektivität für Charybdotoxin-sensitive, calciumabhängige Kalium-Kanäle.

## Claims

1. Cyclohexanediene derivatives of the general formula (Ia,b) 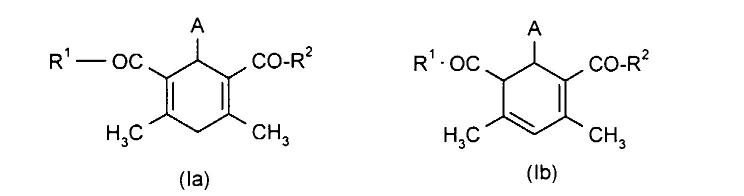 in which
A represents cycloalkyl having 3 to 6 carbon atoms or
represents aryl having 6 to 10 carbon atoms, each of which are optionally substituted up to 3 times, identically or differently, by nitro, cyano, cycloalkyl having 3 to 7 carbon atoms, halogen, trifluoromethyl or by straight-chain or branched alkylthio, alkyl or alkoxy, each having up to 6 carbon atoms,
R¹ and R² are identical or different and
represent hydrogen or straight-chain or branched alkyl or alkoxy each having up to 8 carbon atoms, hydroxyl or represent a group of the formula -NR³R⁴, in which
R³ and R⁴ are identical or different and
denote hydrogen, aryl having 6 to 10 carbon atoms or a straight-chain or branched alkyl having up to 4 carbon atoms,
and their salts.

2. Cyclohexanediene derivatives according to Claim 1, characterized in that
A represents cyclohexyl, phenyl or naphthyl, each of which are optionally substituted up to 3 times, identically or differently, by nitro, cyano, fluorine, chlorine, bromine, iodine, cyclopentyl, cyclohexyl, trifluoromethyl or by straight-chain or branched alkylthio, alkyl or alkoxy each having up to 4 carbon atoms,
R¹ and R² are identical or different and
represent hydrogen or straight-chain or branched alkyl or alkoxy each having up to 6 carbon atoms, hydroxyl or represent a group of the formula -NR³R⁴,
in which
R³ and R⁴ are identical or different and
denote hydrogen, phenyl or straight-chain or branched alkyl having up to 3 carbon atoms,
and their salts.

3. Cyclohexane derivatives according to Claim 1, characterized in that
A represents cyclohexyl or phenyl, each of which are optionally substituted up to 3 times, identically or differently, by nitro, cyano, fluorine, chlorine, bromine, iodine, trifluoromethyl, cyclohexyl, methyl, methoxy or by methylthio,
R¹ and R² are identical or different and
represent hydrogen or straight-chain or branched alkyl or alkoxy each having up to 5 carbon atoms, hydroxyl or represent a group of the formula -NR³R⁴,
in which
R³ and R⁴ are identical or different and denote hydrogen, phenyl, methyl or ethyl,
and their salts.

4. Process for preparing cyclohexanediene derivatives according to any of Claims 1 to 3, characterized in that compounds of the general formula (II) 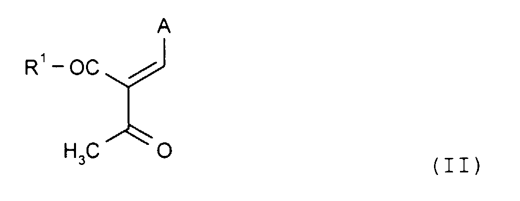 in which
A and R¹ have the meanings stated in Claims 1 to 3 are reacted
with compounds of the general formula (III) 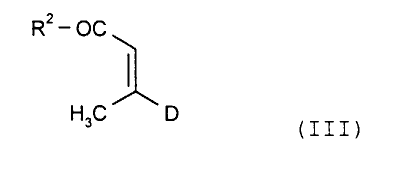 in which
R² has the meanings stated in Claims 1 to 3,
D represents a radical of the formula
CH₂―P(C₆H₅)₃⁺ E⁻
or
CH₂(P(O)(OR⁵)(OR⁶)
in which
R⁵ and R⁶ are identical or different and denote straight-chain or branched alkyl having up to 4 carbon atoms,
E represents chloride or bromide,
in inert solvents, in the presence of a base, and in the case of the acids R¹/R² ≠ OH the esters are hydrolysed,
and in the case of the amides -CO-NH₂, starting from the acids are reacted with ammonia or the appropriate amines (NHR³R⁴),
or starting from the corresponding esters of the cyclohexanediene derivatives of the general formula (Ia,b) an aluminium trimethyl-mediated aminolysis is carried out by the amines (NHR³R⁴) or their hydrochlorides.

5. Cyclohexanediene derivatives according to any of Claims 1 to 3 for therapeutic use.

6. Pharmaceutical containing at least one cyclohexanediene derivative according to any of Claims 1 to 3.

7. Pharmaceutical according to Claim 6 as modulator with selectivity for charybdotoxin-sensitive, calcium-dependent potassium channels.

8. Use of the cyclohexanediene derivatives according to any of Claims 1 to 3 for producing pharmaceuticals.

9. Use according to Claim 8 for producing modulators with selectivity for charybdotoxin-sensitive, calcium-dependent potassium channels.

## Revendications

1. Dérivés de cyclohexanediène de formule générale (Ia,b) : 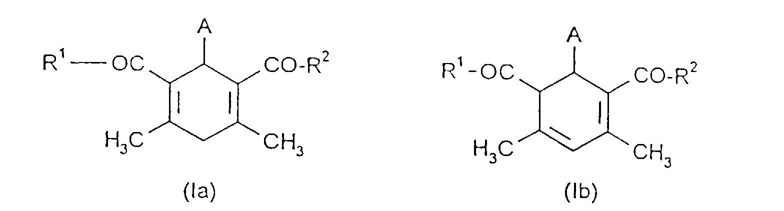 dans laquelle
A représente un groupe cycloalkyle ayant 3 à 6 atomes de carbone, ou bien
un groupe aryle ayant 6 à 10 atomes de carbone, qui sont substitués le cas échéant jusqu'à 3 fois identiques ou différentes par un radical nitro, cyano, cycloalkyle ayant 3 à 7 atomes de carbone, halogéno, trifluorométhyle ou par un radical alkylthio, alkyle ou alkoxy linéaire ou ramifié ayant dans chaque cas jusqu'à 6 atomes de carbone,
R¹ et R² sont identiques ou différents et représentent l'hydrogène ou un groupe alkyle ou alkoxy linéaire ou ramifié ayant dans chaque cas jusqu'à 8 atomes de carbone, un groupe hydroxy ou un groupe de formule -NR³R⁴,
dans laquelle
R³ et R⁴ sont identiques ou différents et représentent l'hydrogène, un groupe aryle de 6 à 10 atomes de carbone, ou un groupe alkyle linéaire ou ramifié ayant jusqu'à 4 atomes de carbone,
et leurs sels.

2. Dérivés de cyclohexanediène suivant la revendication 1, caractérisés en ce que
A représente un groupe cyclohexyle, un groupe phényle, ou un groupe naphtyle, qui sont substitués, le cas échéant, jusqu'à 3 fois identiques ou différentes par un radical nitro, cyano, fluoro, chloro, bromo, iodo, cyclopentyle, cyclohexyle, trifluorométhyle ou par un radical alkylthio, alkyle ou alkoxy linéaire ou ramifié, ayant dans chaque cas jusqu'à 4 atomes de carbone,
R¹ et R² sont identiques ou différents et représentent l'hydrogène ou un groupe alkyle ou alkoxy linéaire ou ramifié ayant dans chaque cas jusqu'à 6 atomes de carbone, un groupe hydroxy ou un groupe de formule -NR³R⁴,
dans laquelle
R³ et R⁴ sont identiques ou différents et représentent l'hydrogène, un groupe phényle ou un groupe alkyle linéaire ou ramifié ayant jusqu'à 3 atomes de carbone,
et leurs sels.

3. Dérivés de cyclohexane suivant la revendication 1, caractérisés en ce que
A représente un groupe cyclohexyle ou un groupe phényle qui sont substitués, le cas échéant, jusqu'à 3 fois identiques ou différentes par un radical nitro, cyano, fluoro, chloro, bromo, iodo, trifluorométhyle, cyclohexyle, méthyle, méthoxy ou par un radical méthylthio,
R¹ et R² sont identiques ou différents et représentent l'hydrogène ou un groupe alkyle ou alkoxy linéaire ou ramifié ayant dans chaque cas jusqu'à 5 atomes de carbone, un groupe hydroxy ou un groupe de formule -NR³R⁴,
dans laquelle
R³ et R⁴ sont identiques ou différents et représentent l'hydrogène, un groupe phényle, méthyle ou éthyle,
et leurs sels.

4. Procédé de production de dérivés de cyclohexanediène suivant l'une des revendications 1 à 3, caractérisé en ce qu'on fait réagir des composés de formule générale (II) : 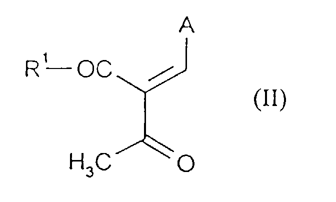 dans laquelle
A et R¹ ont les définitions indiquées dans les revendications 1 à 3,
avec des composés de formule générale (III) : 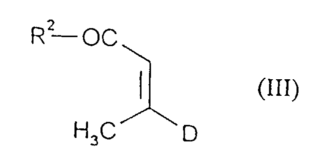 dans laquelle
R² a les définitions indiquées dans les revendications 1 à 3,
D est un reste de formule :
CH₂―P(C₆H₅)₃⁺E⁻
ou
(CH₂-P(O)(OR⁵)(OR⁶)
dans laquelle
R¹ et R² sont identiques ou différents et représentent un groupe alkyle linéaire ou ramifié ayant jusqu'à 4 atomes de carbone,
E est un ion chlorure ou bromure,
dans des solvants inertes, en présence d'une base et, dans le cas des acides R¹/R² ≠ OH, on hydrolyse les esters,
et dans le cas des amides -CO-NH₂, on conduit la réaction à partir des acides avec l'ammoniac ou des amines correspondantes (NHR³R⁴),
ou bien, en partant des esters correspondants des dérivés de cyclohexanediène de formule générale (Ia,b), on conduit une aminolyse par les amines (NHR³R⁴) ou leurs chlorhydrates, par l'intermédiaire de l'aluminium-triméthyle.

5. Dérivés de cyclohexanediène suivant l'une des revendications 1 à 3, destinés à une utilisation thérapeutique.

6. Médicaments contenant au moins un dérivé de cyclohexanediène suivant l'une des revendications 1 à 3.

7. Médicaments suivant la revendication 6, utilisés comme modulateurs doués de sélectivité pour les canaux potassium, sensibles à la charybdotoxine, et dépendant du calcium.

8. Utilisation de dérivés de cyclohexanediène suivant l'une des revendications 1 à 3, pour la préparation de médicaments.

9. Utilisation suivant la revendication 8 pour la préparation de modulateurs doués de sélectivité pour les canaux potassium sensibles à la charybdotoxine, et dépendant du calcium.
